# EUROPEAN PATENT APPLICATION

(11) **EP 1 318 192 A2**
(43) Date of publication of application: **11.06.2003**
(21) Application number: 02014624.7
(22) Date of filing: 02.07.2002
(51) Int. Cl.: C12N 9/10, C12N 15/82

(54) **Prenyltransferase from Arabidopsis**

(30) Priority: 06.12.2001 EP 01129034
(71) Applicant: greenovation Biotech GmbH, 79111 Freiburg i.Br (DE)
(72) Inventor: Schledz, Michael, 79238 Kirchhofen (DE)
(74) Representative: Mechnich, Oliver M.J.

(57) **Abstract**

The present invention relates to the molecular identification of a tocotrienol/plastoquinone prenyltransferase (PQP), which leads to increased tocotrienol-, plastoquinone- and carotenoid-levels in stable transformants of BY-2 tobacco cells.

## Description

Due to their plastids plants possess some biosynthetic pathways, which are, besides in cyanobacteria, unique in living organisms. Some plastidic compounds are indispensable for human and animal nutrition and are therefore called vitamins.Two essential lipophilic components for nutrition are provitamin A (beta-carotene) and vitamin E.

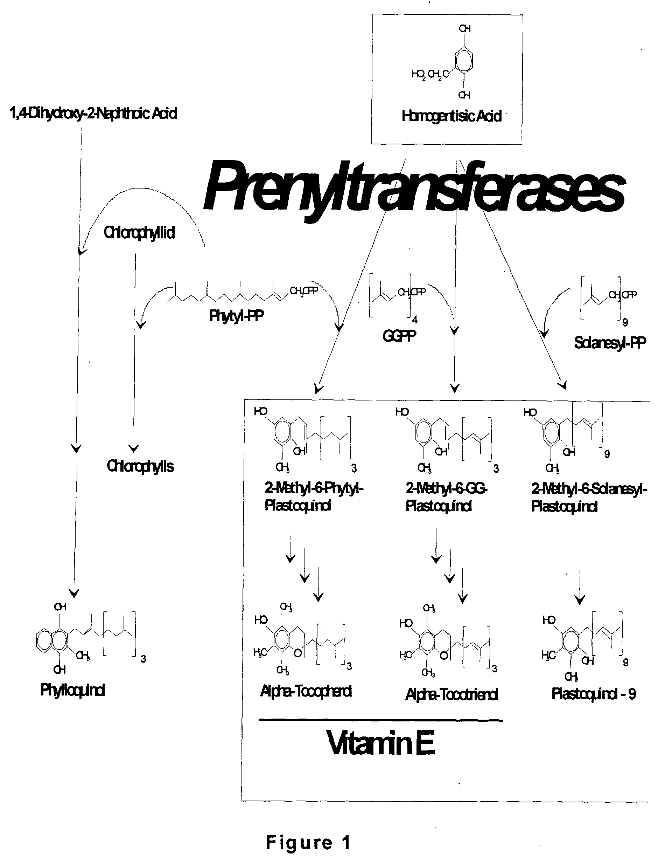

Vitamin E is predominantly delivered by the ingestion of vegetable oils. It plays an important role as a membrane-associated antioxidant scavenger (Brigelius-Flohe and Traber, 1999). During past years several additional functions of vitamin E as antihypercholesterolemic and immunostimulatory agent in humans have been proposed (Beharka *et al.,* 1997). Vitamin E is classified by its pharmacological effect and chromanol ring structure and not by biosynthesis. It comprises a class of 8 lipid-soluble components, being subdivided into tocopherols and tocotrienols (Fig. 1).

While tocopherols share an isoprenoid side chain derived from phytyl-PP, tocotrienol side chains are derivates of geranylgeranyl-PP. The α, β, γ and δ-members of these subclasses differ in their degree of methylation in the 6-chromanol-ring structure. Especially tocotrienols are discussed to have a higher potency in protection of eucaryotic cell membrane oxidation (Jandak *et al.,* 1989; Kamat *et al.,* 1997). Moreover, tocotrienols have been shown to suppress cholesterol biosynthesis by posttranscriptional inhibition of hydroxymethyl glutaryl-CoA-reductase. They are involved in the degradation of artherogenic apolipoproteine B and the reduction of lipoprotein plasma levels (Parker *et al.,* 1993; Wang *et al.,* 1998), whereas tocopherols don't have these pharmacological effects (Qureshi *et al.,* 1995). In addition, tocotrienols have been suggested to have an anti-thrombotic and anti-tumor effect indicating that they may serve as an effective agent in the prevention or treatment of cardiovascular disease and cancer (Theriault *et al*., 1999). From the pharmacological point-of-view, the current formulation of vitamin E supplements, which is comprised mainly of α-tocopherol, may be questionable.

In plastids many isoprenoid pathways are localized, which are interconnected by their substrates, end products and by regulation. These are,e.g. monoterpene-, diterpene-, giberillic acid-, abscisic acid-, chlorophyll-, phylloquinone-, carotenoid-, tocopherol-, tocotrienol- and plastoquinone-biosynthesis. In all these pathways different prenyltransferases are involved in the biosynthesis of these compounds. With respect to the length of their side chains diterpenes, chlorophylls, phylloquinones, tocopherols and tocotrienols can be arranged into a C₂₀-group of isoprenoids.

Another classification by degree of desaturation of the side chain, would arrange e.g. chlorophylls, phylloquinones and tocopherols into a phytyl-group and e.g. diterpenes, tocotrienols, plastoquinones and carotenoids into a group of desaturated isoprenoid compounds. Classification by head groups would arrange tocopherols, tocotrienols and plastoquinones (Fig.1) in one group, being quinones with antioxidant properties and having homogentisic acid as a precursor. Plastoquinones are important components of the quinone-pool in the photosynthetic electron transport chains of plastids, also interfering in the biosynthesis of provitamin A (beta-carotene; Norris *et al*., 1995). Due to the interconnections you have to speak of an isoprenoid network in plastids, where you can only address the question of a biosynthetic pathway in terms of substrate specificity of an enzyme.

We addressed the question for the prenyltransferase-reaction in tocotrienol- and plastoquinone-biosynthesis.

The pathways of tocopherol, plastoquinone and tocotrienol biosynthesis have not been well characterized. Although many activities have been measured *in vitro* using radiolabelled precursors, the functional characterization of individual enzymes was severely hindered by difficulties in their purification. This was mainly due to their membrane-bound localization and to their low abundance *in vivo.*

Focussing on the vitamin E biosynthesis 3 genes, coding for non-prenyltransferases, have been cloned and functionally characterized from *Arabidopsis,* i.e. *p*-hydroxyphenylpyruvate-dioxygenase, GGPP-reductase and γ-tocopherol methyltransferase (Keller *et al*., 1998; Norris *et al*., 1998; Shintani *et al*., 1998).

Prenyltransferases involved in the formation of tocopherols and tocotrienols/plastoquinones are expected to mark a branching point of these pathways (Fig.1). These enzymes are supposed to catalyze the condensation of saturated/desaturated isoprenoid-diphosphates with homogentisic acid by a complex prenylation/phytylation-reaction involving decarboxylation of the aromatic moiety.

With regards to the literature a *pds*2-mutant has been described being deficient in plastoquinones and impaired in the carotenoid level (Norris *et al.,* 1995). This mutant was found in a screening assay of Arabidopsis mutants that disrupt the plastoquinone-dependent desaturation of carotenoids and lead to the accumulation of phytoene, the first carotenoid in this pathway. The undetectable amounts of tocopherols in the mutant, perhaps being a consequence of the identification by absorbance and not by more sensitive fluorescence, arose the question of a multifunctional phytyl/prenyl transferase for plastoquinone, tocotrienol and tocopherol biosynthesis.

The assumption had to be revised: a multifunctional prenyltransferase, accepting phytyl-PP, geranylgeranyl-PP and solanesyl-PP as substrates for tocopherol, tocotrienol and plastoquinone biosynthesis (Fig. 1) does not exist.

We identified a tocopherol phytyltransferase from Synechocystis sp.PCC 6803, taking advantage of the fact that the quinone pattern of the cyanobacterium is similar to that of plants. Analyzing the deletion mutant of *slr1736* by its loss of function, it was impaired in tocopherol content. However plastoquinone and carotenoid levels remained unchanged (Schledz *et al.,* 2001). Performing a FASTA- alignment of SLR1736 peptide sequence (CyanoBase; Kazusa DNA Research Institute; http://www.kazusa.or.jp) against the genomic database of Arabidopsis thaliana (tfastx3_t; TAIR: The Arabidopsis Information Resource; http://www.arabidopsis.org; Pearson and Lipman, 1988) highest homology (51,22 % identity in a 41 aminoacids overlap) lightens up to a hypothetical protein, coded by BAC-clone F19F24.15 and located on chromosome 2. With some corrections to the exon/intron-structure, cloning of the cDNA and recombinant expression of the protein it could be currently demonstrated by *in vitro*-enzymatic assays with radioactive precursors that this protein is the tocopherol phytyltransferase of *Arabidopsis*. In these assays specificity for GGPP or solanesyl-PP of Arabidopsis tocopherol phytyltransferase was not detectable (Collakova *et al*., 2001).

The authors assumed that a different prenyltransferase has to exist that catalyzes the prenylation reaction of plastoquinone biosynthesis and that the tocopherol phytyltransferase catalyzes the formation of tocopherols. Tocotrienols are not mentioned in this context.

In contrast, we assumed that a prenyltransferase exists in higher plants which is compromising their isoprenoid substrates with respect to their saturation but independent of their chain length. We think that the vitamin E group is a historical relict and that the prenylation/phytylation reactions for tocotrienols and for tocopherols are performed by two different enzymes. We demonstrate, that the prenylation reaction in the biosynthetic pathways of tocotrienols and plastoquinones is catalyzed by the same enzyme. We assume that their biosynthetic branching point is the cyclization of the chromanol ring structure, mediated by tocopherol cyclase. *In vitro* data suggest that the cyclization of 2-methyl-6-geranylgeranyl-plastoquinone to γ-tocotrienol, i.e. condensation reaction to the chromanol ring structure, seems to be performed by the same tocopherol cyclase in cyanobacteria (Stocker et al., 1996). We assume that this cyclase is isoprenoid chain length specific, irregardless of the saturation degree of their substrates, and is discriminating other chain lengths than C₂₀. This is the biosynthetic branching point where tocotrienols and other plastoquinones split.

We report here on the molecular identification of a tocotrienol/plastoquinone prenyltransferase (PQP), which leads to increased tocotrienol-, plastoquinone- and carotenoid-levels in stable transformants of BY-2 tobacco cells.

### 2. Results

### 2.1 Arabidopsis database analysis for tocotrienol / plastoquinone prenyltransferase.

*Pds2* mutation, leading to a deficiency in plastoquinones, was mapped to the top of chromosome 3, ∼7 centimorgans above long hypocotyl2 (hy2) in *Arabidopsis* (Norris *et al.,* 1995; classical map). The genomic sequence of the described tocopherol phytyltransferase, which cannot synthesize tocotrienols and plastoquinones *in vitro,* can be located on chromosome 2 (see above; Collakova *et al*., 2001).

Due to the performed similar decarboxylation/prenylation reaction and similar substrates the peptide sequence of SLR1736 was utilized to search for tocotrienol/plastoquinone prenyltransferase in *Arabidopsis* databases.

However, strategy was different than directly searching for homologues in the genomic Arabidopsis database. First, we looked in an Arabidopsis EST-database (arabiEST) for deduced peptide sequences of expressed sequence tags being homologous to the peptide sequence of SLR1736. Some of them showed significant homologies,e.g. AV521188 (58/97 positives), AV535532 (19/24 positives), AV535920 (19/24 positives). The cDNA-sequence of these ESTs was aligned to the genomic Arabidopsis database looking for 100 % identities in stretches longer than 100 bp.

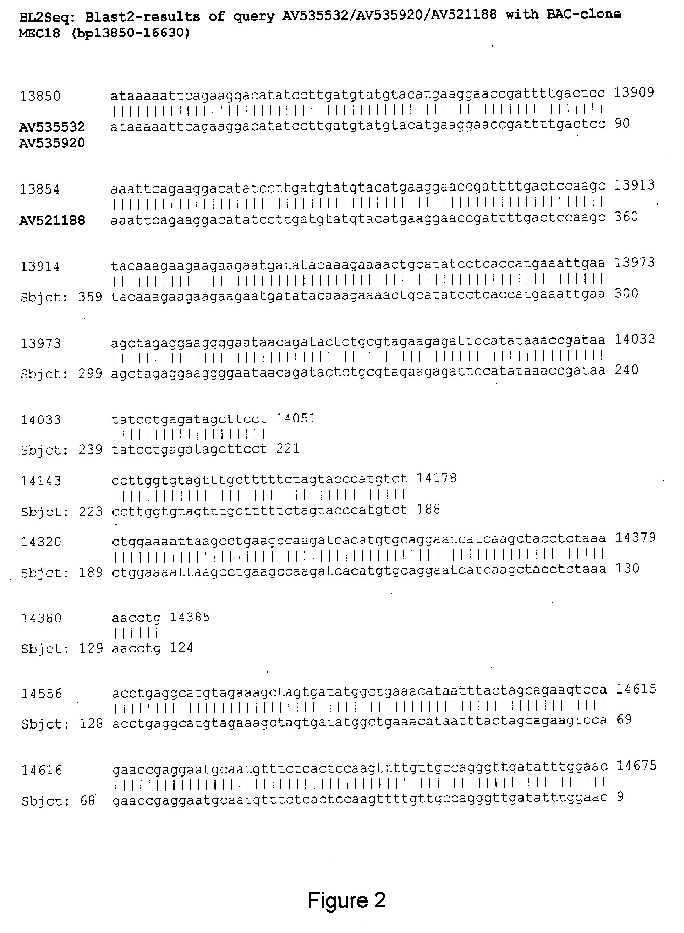

Application of this strategy to the already named ESTs lead to the identification of the genomic sequence. These ESTs map to BAC clone F26K24 (accession no. AC016795), not being recognized as part of a coding sequence. Sequence is identical to Arabidopsis genomic DNA, P1 clone MEC18 (acession no. AP002040; Nakamura, 2000). The predicted coding sequence of MEC18.5 is only partly correct as the exon/intron borders are set falsely. In Fig. 2 some details of the Blast2-results of the alignments of these ESTs with genomic DNA of MEC18.5 (MEC18; bp13850-16630) are shown. F26K24 can be localized on chromosome 3 (AGI map and Altmann map), the same chromosome where *pds*2-mutation was mapped. A comparison with the classical map, including *pds2* as a gene marker for recombination is difficult, as there is no direct conversion of centimorgans into base pairs.

### 2.2 Cloning of tocotrienol/ plastoquinone prenyltransferase

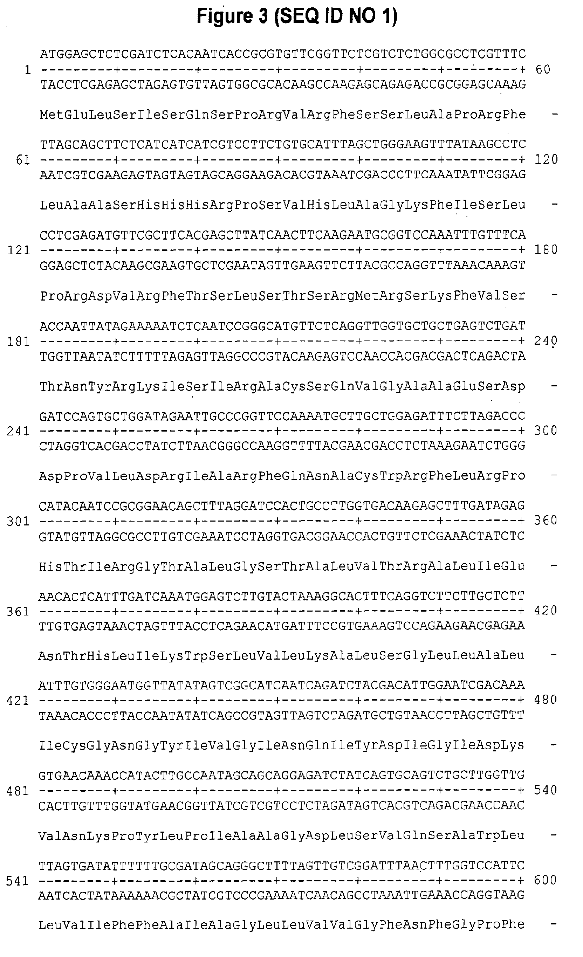

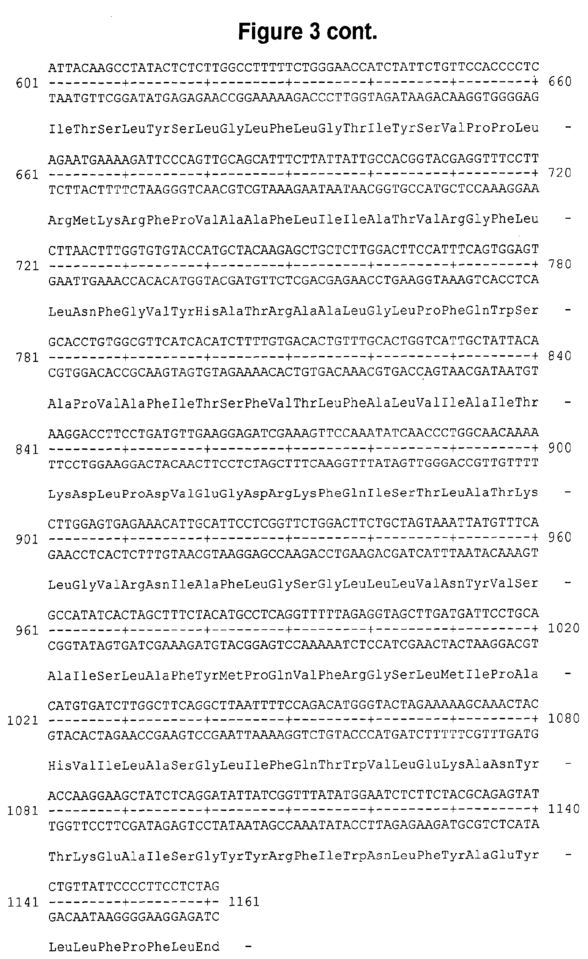

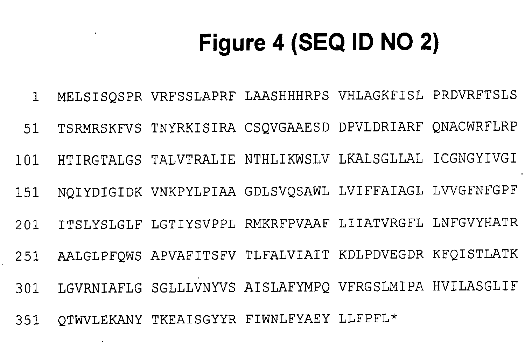

The tocotrienol/plastoquinone prenyltransferase-cDNA was amplified from an *Arabidopsis* Matchmaker cDNA library (Clontech, Germany) by using the primers *PQ5* (SEQ ID NO 3) and *PQ3* (SEQ ID NO 4). Primer selection of *PQ3* could be performed as the depicted EST clones were coding for the complete 3'-coding region. Primer *PQ5* was selected by the assumption that the first recognized exon in the genomic *Arabidopsis* database starts with the correct Start-ATG. The purified PCR-product was cloned into *E.coli* expression vector pBADTOPO. Two independent clones, PQBAD1 and PQBAD2, were sequenced and no differences between them could be detected. The 1161 bp coding sequence of tocotrienol/plastoquinone prenyltransferase (Fig. 3; SEQ ID NO 1) codes for a deduced protein of 386 amino acids and ∼42.84 KDa (Fig.4; SEQ ID NO 2).

### 2.3 Alignments, homologies and prenyltransferase domains

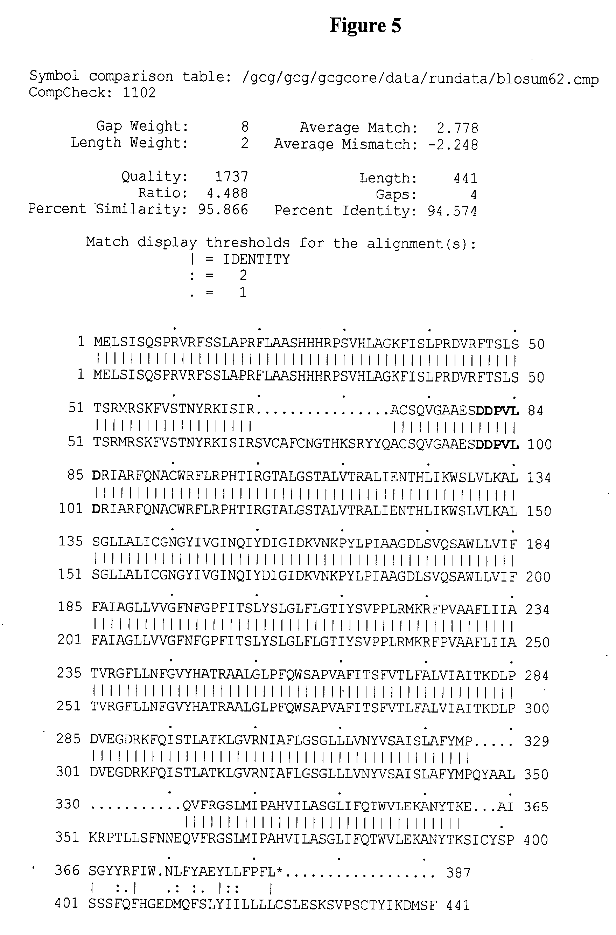

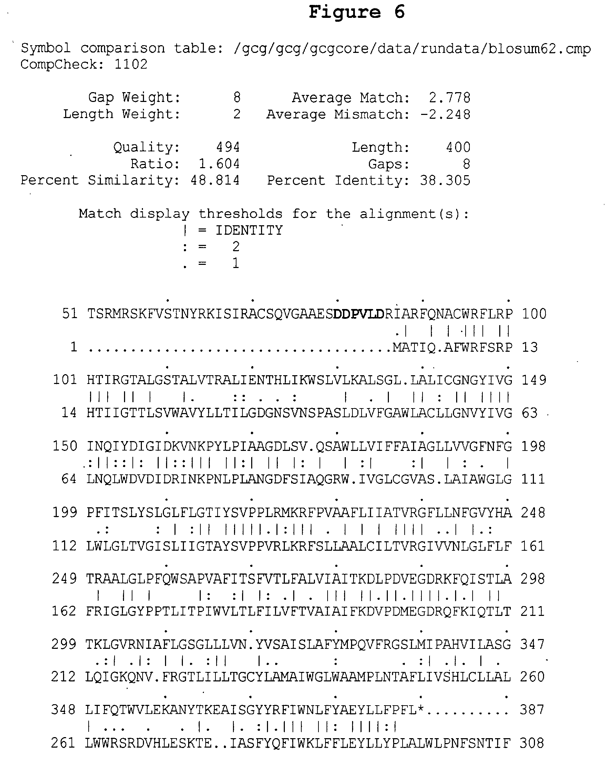

In Fig.5 a GAP-alignment between the protein-sequence of tocotrienol/plastoquinone prenyltransferase and the predicted protein-sequence of MEC18.5 has been performed, also giving the parameters of the alignment. It shows clearly that the inconsistencies due to false predicted intron/exon borders are at the N-terminal end of the protein directly in front of the potential prenyltransferase domain (GAAES**DDPVLD**RIARFQ; aa position 75-91 of SEQ ID NO 2), the so called DDXXD-motive of prenyltransferases, and at the C-terminal part in a range of 96 amino acids (amino acids 346 to 441 of the predicted protein). This motive of apartate (D) or glutamate(E)-residues is known to bind cations (preferentially Mg²⁺ and Mn²⁺) to form a complex with the diphosphate moiety of isoprenoid diphosphates in the reaction center. Taken all inconsistencies of the predicted protein into account 17.9 % of the amino acids didn't match (79 aa/441 aa). Comparing the peptide-sequences of tocopherol/tocotrienol phytyltransferase with tocopherol phytyltransferase of *Synechocystis* (SLR1736) in the same GAP-program only 38.3% identity/ 48.8% similarity between them can be observed (Fig. 6). The putative prenyltransferase domain (GAAES**DDPVLD**RIARFQ; aa position 75 to 91 in PQP (SEQ ID NO 2)) is not recognized as a homologous sequence, as alignment by this program starts at aa 87 of SEQ ID NO 2 (IARFQ). Putative prenyltransferase domain of *Synechocystis* is located at aa 60 to 83 (YIVGLNQLW**DVDID**RINKPNLPLA; Schledz *et al.,* 2001; Collakova *et al*., 2001). This sequence can be aligned to aa 146 to 169 in PQP (SEQ ID NO 2; YIVGINQIY**D**IGI**D**KVNKPYLPIA), where the classical motive of DDXXD is hardly detectable.

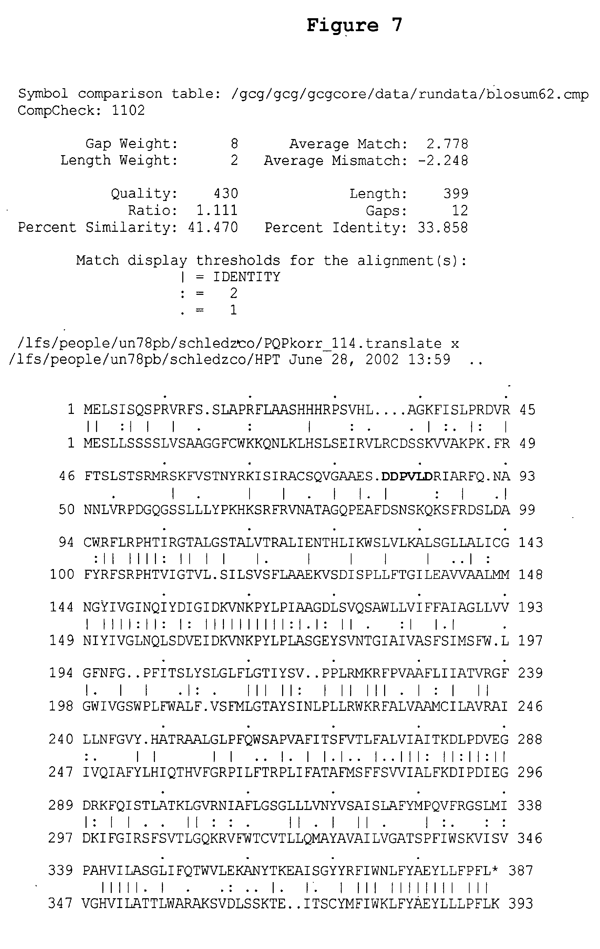

Protein-sequences of tocotrienol/plastoquinone prenyltransferase and tocopherol phytyltransferase of *Arabidopsis* were aligned by the GAP-program (Fig. 7). They have only 33.8 % identity/41.4 % similarity, revealing very low similarities in the depicted putative prenyltransferase-domain of PQP (aa 75 to 91 in SEQ ID NO 2).

The protein-sequence of PQP was aligned in a TBLASTN2.2.1 against the available nucleotide libraries, excluding ESTs, STS, GSS or phase 0, 1 or 2 HTGS sequences. Signifikant identities could only be observed to the already cited genomic *Arabidopsis* BACs F26K24, MEC18 and to *slr1736* of Synechocystis. Other sequences showing some homology were predominantly coding *chl*G-genes or bacteriochlorophyll-genes, as *slr*0056 (chlorophyll synthase chlG) of *Synechocystis.*

### 2.4 Transformation of BY-2 tobacco cell suspension culture and analysis of transformants

For stable transformation and overexpression in BY-2 tobacco cells PQP- cDNA was cloned under the control of the constitutive Cauliflower Mosaic Virus (CaMV) 35S-promoter in agrobacterial binary vector pC1390PQ-35S. Transformation and propagation of BY-2 cell suspensions, as well as passages of cultures were performed as written in Material and Methods. Individual wild-type and transformed cell cultures were harvested and extracted at the same day. Analysis was three times repeated for individual cultures to diminish the effect of putative environmental factors (table 1, table 3, table 5). Wild type as transformants grew normally. However, transformants showed a yellowish colour compared to the wild-type, indicating the presence of carotenoids. Prior to extraction dried cell cultures were internally standardized by the addition of α-tocopherol acetate (for vitamin E; fluorescence detection) and echinenone (for carotenoids; detection by absorbance).

With respect to vitamin E the major compound that accumulates in wild-type BY-2 cells is α-tocotrienol.The only other vitamin E compound which can be identified by its fluorescence and retention time in extracts of BY-2 cells is δ-tocopherol (table 1).

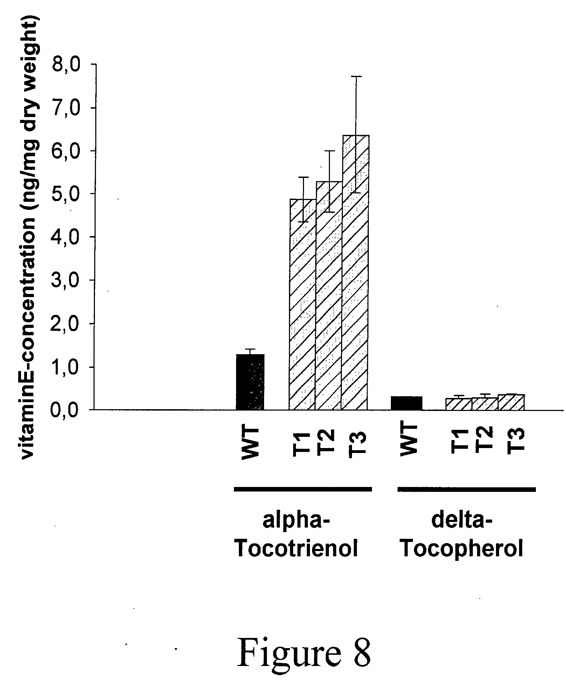

As would be expected by transformants overexpressing tocotrienol/plastoquinone prenyltransferase an overall increase in α-tocotrienol levels can be observed (300-500 %; table 2; Fig. 8). In contrast, tocopherol levels are not changed significantly in transformants (88-116 %; table 2).

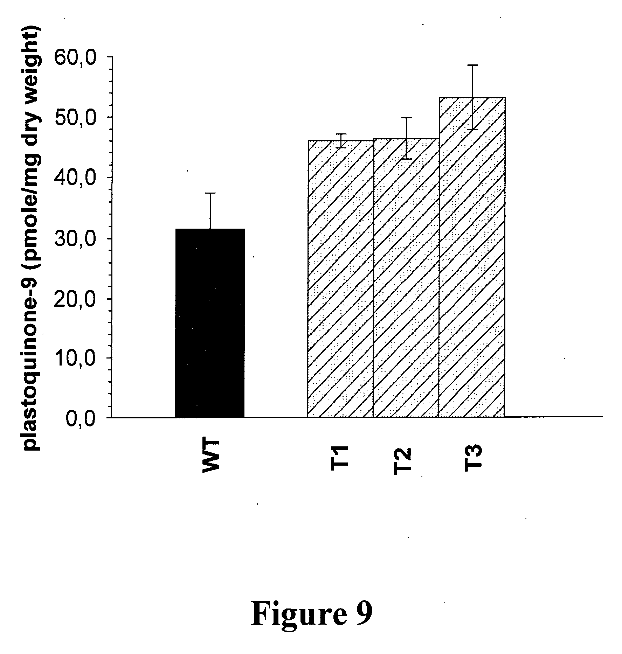

We also analyzed the plastoquinone-9 content of wild-type and BY-2 cells transformed with pC1390PQ-35S (table 3). Therefore we externally standardized the extracts by the utilization of decyl-plastoquinone. The retention time of plastoquinone-9 in our HPLC-system (60. min) was assayed with a plastoquinone-9 standard prepared from spinach leaves (see Material and Methods). In transformants plastoquinone-9 levels were increased to 146-169 % compared to the wild-type (table 4; Fig. 9).

Carotenoids that predominantly accumulate in BY-2 cells are xanthophylls (i.e. violaxanthin, antheraxanthin, neoxanthin and their esters) and β-carotene. For quantification of the overall carotenoid content an Eₘ =133, 000 was assumed.

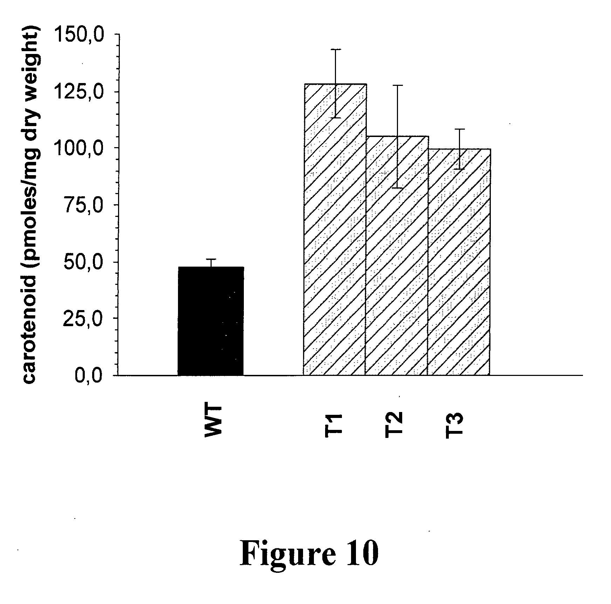

As observed with tocotrienols and plastoquinones carotenoid levels (table 5) were increased in transformants relative to the wild-type (209-215 %; table 6; Fig. 10).

Summarizing BY-2 cells transformed with pC1390PQ-35S have increased levels of tocotrienols, plastoquinones and carotenoids.

### 3. Discussion

An increase in different plastidic isoprenoid pathways can be explained by two different hypothesis:
1. The overexpression of a gene product leads to the accumulation of a compound, which is the prerequisite for different isoprenoid pathways (e.g. isopentenyl diphosphate for all isoprenoid compounds).
2. The overexpression of a gene product leads to the accumulation of a compound, which is the prerequisite for only one isoprenoid pathway. Accumulation of the biosynthetic end product or intermediates of this pathway cross-talk with enzymes of other isoprenoid pathways and influence their regulation.

We believe, that the second hypothesis hold true in our case. We isolated a prenyltransferase of *Arabidopsis* with homologies to prenyltransferases, located at the branching point of different isoprenoid biosynthetic pathways. As tocotrienols and plastoquinones are upregulated in transformants overexpressing this gene product, one can conclude that this prenyltransferase catalyzes the condensation reaction of desaturated isoprenoid-diphosphates with homogentisic acid irregardless of the chain length. Carotenoids are also upregulated. Since the desaturation of phytoene in carotenoid biosynthesis is dependent on a NaDPH-dependent quinone oxidoreductase (Mayer *et al.,* 1992) and the plastoquinone-pool in plastidic membranes (Norris *et al*., 1995) upregulation can be explained by the upregulation of quinone-acceptors (tocotrienols, plastoquinones) in these membranes. Our data is consistent with the effects described by a *pds*2-mutant in *Arabidopsis,* where decreased plastoquinone-levels and accumulation of phytoene can be measured (Norris *et al*., 1995). For homogentisic acid phytyltransferase (HPT) of *Arabidopsis,* referred as tocopherol phytyltransferase, no changes in plastoquinone- and carotenoid- levels are observed. Direct evidence comes from loss-of function mutants in *Synechocystis* (Schledz *et al*., 2001) and *in vitro*-enzymatic assays with HPT from *Arabidopsis* (Collakova *et al.,* 2001). Additionally, indirect evidence with *Nicotiana tabacum* and *Arabidopsis,* stably transformed with an *anti-sense* expression cassette of geranyl geranyl diphosphate reductase (*ggpr*), indicates the existence of a tocotrienol prenyltransferase: In these transformants only decreased chlorophyll- and tocopherol-levels can be observed (Tanaka *et al*., 1999), whereas there is a significant increase in tocotrienols. In 17 days old wild-type *Arabidopsis* plants without flowers 388.4 +/- 97.0 ng tocopherols/mg dry weight can be measured, no tocotrienols are detectable. Whereas in the T3-generation of 17 days old *anti-sense ggpr* transgenic *Arabidopsis* only eg. 155.1 +/- 34.6 or 134.6 +/- 29.7 ng vitamin E/mg dry weight can be measured. However the amount of tocotrienol in this vitamin E-pool increases from 0 % to e.g. 6.3 % or 20.0 % compared to the wild-type. In these transformants also carotenoid-concentrations are decreasing from 100 % to eg. 41.4 or 38.7 % compared to the wild-type. All these observations are consistent with our identification of a tocotrienol/plastoquinone-prenyltransferase in *Arabidopsis thaliana.*

### 4. Material and Methods

### 4.1 Cloning procedures

A 1193 bp-fragment, representing the 1161 bp coding sequence of the tocotrienol/plastoquinone prenyltransferase-cDNA and additional 32 bp, was amplified from 1 µg of an *Arabidopsis* Matchmaker cDNA library (Clontech, Germany) using the primers *PQ5* (5'-AAGCTTCTGGAGCTGTCGATCTCACAATCACCGCGT-3'; SEQ ID NO 3) and *PQ3* (5'-CATATCGTCGACATGAAATTGAAAGCTAGAGGAAGG-3'; SEQ ID NO 4). PCR with Taq-polymerase (Amersham Pharmacia Biotech, Germany) was carried out after a denaturation of 3 min at 94°C for 30 cycles (60 s at 94°C, 60 s at 60°C, 80 s at 72°C), followed by an extension at 72°C for 10 min. Thereby a *Sall*-site was generated in the 3'-non coding region of the PCR-product, as well as the start ATG-codon (to CTG) and a *Sacl*-site (to GAGCTG) modified in the coding sequence for latter cloning strategies. The modification in the *Sacl*-site leads to a conserved *Leu to Leu* displacement in the deduced amino acid sequence of the open reading frame.The purified PCR-product (GFX PCR and Gel Purification Kit; Amersham Pharmacia Biotech, Germany) was cloned into the *E.coli* expression vector PBADTOPO TA (Invitrogen, the Netherlands). Two independent clones in different orientations to the arabinose-promoter (PQBAD1 and PQBAD2) were sequenced and no differences between them could be detected. The 1161bp-coding sequence was compared to the sequence of *Arabidopsis* genomic DNA (chromosome 3; P1 clone MEC18) by the GAP-program (GCG software package) and the correct exon/intron-structure could be achieved.

For the stable transformation of a BY2-tobacco cell culture, the prenyltransferase-cDNA with a correct Start-ATG was amplified from the vector PQBAD2 by proof-reading PCR with AdvanTaq-Polymerase (Clontech, Germany) using the primer pair *PQBY5* (5'-GCCCTTCCCGGGATGGAGCTGTCGATCTCACAATCA-3'; SEQ ID NO 5) and *PQBY3* (5'-TGAAATGAATTCCTAGAGGAAGGGGAATAACAGATA-3'; SEQ ID NO 6). After denaturation for 3 min at 94°C, 30 cycles (94°C for 30s/60°C for 45s/68°C for 120 s) and an extension (68°C for 5 min) were performed. The purified PCR-product was digested *SmaI*/ *EcoRI* and cloned into the agrobacterial binary vector pC1390-35S, digested with the same enzymes. pC1390-35S is a derivative of pCambia1390 (accession no. 234307), consisting of an additional 35CaMV 35S-promoter, integrated clockwise in between the *PstI*/*BamHI*-sites of this vector. The resulting vector pC1390PQ-35S was transformed into BY-2 tobacco cells.

### 4.2 Direct DNA transfer in Agrobacterium tumefaciens

*Agrobacterium tumefaciens* strain GV3101 (pMP90RK) was grown for 3 days on LB-plates containing 50 µg/ml rifampicin and 25 µg/ml gentamycin at 28°C. A single colony was inoculated in 5 ml LB-medium containing the described antibiotics and grown over night at 28°C with shaking (130 rpm). For the generation of chemocompetent bacteria 4 ml over night culture were added to 100 ml LB-medium with 50 mg/ml rifampicin and 25 µg/ml gentamycin and grown as above until the culture reached an OD₆₀₀ of 0.5 to 1.0. The culture was chilled on ice, afterwards the cell suspension was centrifuged for 5 min at 3000 *g* at 4°C. The supernatant was discarded and the cells resuspended in 1 ml 20 mM CaCl₂ solution (ice-cold). 0.1 ml aliquots were dispensed into prechilled Eppendorf test tubes. For transformation 1 µg of vector pC1390PQ-35S was added to the chemocompetent cells and immediately frozen in liquid nitrogen. The cells were thawed by incubating the test tube in a 37°C water bath for 5 min. Afterwards 1 ml of prewarmed LB-medium was added to the tube and the cells incubated for 4 h at 28°C with gentle shaking. Cells were isolated by a 30 sec-centrifugation, resuspended in 0.1 ml LB-medium and spread on LB-plates containing 25 µg/ml gentamycin and 20 µg/ml kanamycin. Transformed colonies appeared after 2 to 3 days. They were inoculated in LB-medium containing 50 µg/ml rifampicin, 25 µg/ml gentamycin and 20 µg/ml kanamycin and transformation verified by colony-PCR (Sambrook *et al*., 2001).

### 4.3 Transformation and propagation of BY-2 cell suspension culture

A *Nicotiana tabacum* BY-2 cell suspension culture (Nagata *et al*., 1992) was grown in MS culture medium (1x MS salts, pH5.8; 0.2 g/l KH2PO4; 30 g/l sucrose; 0.2 mg/l 2,4-D (auxine); 1 mg/l thiamine-HCI; 0.1 g/l *myo*-inositol) at 28°C and 130 rpm in the dark. The culture was subcultured weekly by transferring 2 ml of culture into 100 ml fresh media.

For transformation, 1 ml of cell culture from day 6 *post* inoculation was mixed with 12 ml MS medium and 200 µl of *agrobacterium tumefaciens* strain GV3101 (pMP90RK), transformed with vector pC1390PQ-35S. After 4 days of incubation at 28°C in the dark cells were isolated by centrifugation (5 min at 2000 *g* and room temperature), cell pellets washed twice with culture medium containing 125 µg/ml β-bactyl (Smithkline Beecham, Germany). Afterwards the cells were transferred into 50 ml fresh culture medium containing 125 µg/ml β-bactyl and 30 µg/ml hygromycin.

Reaching the stationary phase 1 ml of culture was transferred into fresh medium with additives as above. Transfer was repeated once before analysis of the transformants was performed.

### 4.4 Extraction of BY-2 cell suspension cultures

Wildtype BY-2 cells and those stably transformed with vector pC1390PQ-35S were harvested 7 days after subcultivation. Medium was removed by filtration through nylon mesh and cells lyophilized to dryness. After the determination of dry weight cells were resuspended in 35 ml of acetone. For internal standardization 40 µg of D-α-tocopherol acetate (Sigma, Germany) and 2 nmoles of echinenone, purified from *Synechocystis sp.PCC6803,* were added. Soluble compounds were extracted twice by harsh sonication on ice followed by centrifugation (10 min, 6000 *g*, 4°C).

Remaining cell debris in the extracts was removed by filtration through a 0.2 µm membrane filter (Schleicher & Schuell, Germany) under vacuum. Pooled acetone phases were dried by rotary evaporation and compounds dissolved in 2 ml of chloroforme. After evaporation compounds were solubilized in 500 µl chloroforme and transferred to eppendorf tubes. Again, extracts were evaporated to dryness.

Then compounds were solubilized in 50 µl chloroforme and subjected to HPLC-analysis. For the determination of lipophilic compounds wildtype BY-2 cells and three transformants were analyzed. To diminish errors in subcultivation and extraction 3 different subcultures of wildtype and transformants were cultivated at different time points and analyzed.

### 4.5 Quantification

Decyl-plastoquinone (Sigma, Germany) was quantified spectrophotometrically at 255 nm prior and after reduction with NaBH₄ in absolute ethanol ( Δ Eₘ = 15,000) (Barr and Crane, 1971), 15 nmoles were used for calibration. D-α-tocopherol acetate (Sigma, Germany) was reduced with NaBH₄ and measured in absolute ethanol at 284 nm (E^{1%} _{cm} = 45; value given by the manufacturer). Tocopherol - and tocotrienol-standards (Merck, Germany) were quantified as described (E^{1%} _{cm} see Schuep and Rettenmaier, 1994) and also analyzed in the form of their oxidized quinones after treatment with 200 µM potassium ferricyanide.

For calibration the absorption/emission peak areas of the quantified standards were analyzed by HPLC. Tocopherols and tocotrienols were detected by use of a fluorescence detector setting excitation and fluorescence emission to 290 nm and 324 nm (40 nm band width), respectively . All other standards were calibrated at their maximal absorbance.

The tocopherol content in the samples was determined by internal standardization adding 40 µg D-α-tocopherol acetate to the lyophilized bacterial pellet prior to extraction. Likewise, carotenoid content was determined by internal standardization with 2 pmoles echinenone. Quantification of echinenone-standard was performed spectrophotometrically at 461 nm (Eₘ =118,900). In BY-2 extracts predominantly violaxanthin, antheraxanthin, neoxanthin, β-carotene and their respective esters can be detected. To quantify the overall content of carotenoids an Eₘ = 133,000 was assumed.

Plastoquinone was quantified by external standardization with decyl-plastoquinone (Sigma, Germany).

To identify plastoquinones in BY-2 cells, a plastoquinone-9 standard from spinach leaves was prepared by acetone-extraction (Barr and Crane, 1971). It was purified by TLC (silica gel 60, Merck, Germany) using petroleum benzene/diethylether (7:1; v:v) (Soil and Schultz, 1979).

### 4.6 HPLC-analysis

The HPLC-system (Waters, Germany) consisted of two 510 HPLC pumps, a 717plus autosampler, a 996 photodiode array detector monitoring UV/VIS spectra, a 474 scanning fluorescence detector and a C₃₀-reversed HPLC column (YMC Europe, Germany). Chromatograms were analyzed using the Millenium PDA software package (Waters, Germany). The column was developed at a flow rate of 1 ml/min with the solvent system A: methanol/*tert*-butylmethyl ether/water (60:12:12; v:v:v) and B: methanol/ *tert*-butylmethyl ether (50:50; v:v). A linear gradient was performed from 100 % A to 57 % A in 45 min, followed by a linear gradient to 0 % A in 35 min and an isocratic step at 0 % A for 25 min. 50 µl of chloroforme extracts were injected.

### References

**Barr R and Crane FL** (1971). Quinones in algae and higher plants. *Methods Enzymol.* **13,** 372-408.

**Beharka A, Redican S, Leka L and Meydani SN** (1997). Vitamine E status and immune function. *Methods Enzymol*. **282,** 247-263.

**Brigelius-Flohe R and Traber MG** (1999). Vitamin E: function and metabolism. *FASEB J*. **13,** 1145-1155.

**Collakova E and DellaPenna D** (2001). Isolation and functional analysis of homogentisate phytyltransferase from *Synechocystis* sp. PCC 6803 and Arabidopsis. *Plant Physiol*. **127,** 1113-1124.

**Jandak J, Steiner M and Richardson PD** (1989). Alpha-tocopherol, an effective inhibitor of platelet-adhesion. *Blood* **73,** 141-149.

**Kamat JP, Sarma HD, Devasagayam TPA, Nesaretnam K and Basiron Y** (1997). Tocotrienols from palm oil as effective inhibitors of protein oxidation and lipid peroxidation in rat liver microsomes. *Mol. Cell Biochem.* **170,** 131-138.

**Keller Y, Bouvier F, D'Harlingue A and Camara B** (1998). Metabolic compartmentation of plastid prenyllipid biosynthesis. Evidence for the involvement of a multifunctional geranygeranyl reductase. *Eur. J. Biochem*. **251,** 413-417.

**Mayer MP, Nievelstein V and Beyer P** (1992). Purification and characterization of a NADPH dependent oxidoreductase from chromoplasts of *Narcissus pseudonarcissus:* a redox mediator possibly involved in carotene desaturation. *Plant Physiol. Biochem.* **30,** 389-398.

**Nagata T, Nemoto Y and Hasezawa S** (1992). Tobacco BY-2 cell line as the "HeLa" cell in the cell biology of higher plants. *Int. Rev. Cytol.* **132,** 1-30.

**Nakamura** Y. (2000). Structural analysis of Arabidopsis thaliana chromosome 3. II.

Sequence features of the regions of 4,251,695 bp covered by ninety P1, TAC and BAC clones. *DNA Res.* **7**, 217-221.

**Norris SR, Barrette TR and DellaPenna** D. (1995). Genetic dissection of carotenoid synthesis in *Arabidopsis* defines plastoquinone as an essential component of phytoene desaturation. *Plant Cell* **7**, 2139-2149.

**Norris SR, Shen X and DellaPenna** D (1998). Complementation of the Arabidopsis *pds1* mutation with the gene encoding *p*-hydroxyphenylpyruvate dioxygenase. *Plant Physiol*. **117,** 1317-1323.

**Parker RA, Pearce BC, Clark RW, Gordon DA and Wright JJK** (1993). Tocotrienols regulate cholesterol production in mammalian cells by posttranscriptional suppression of 3-hydroxy-3-methylglutaryl-coenzyme A reductase. *J. Biol. Chem.* **268,** 11230-11238.

**Pearson WR and Lipman DJ** (1988). Improved tools for biological sequence comparison. *Proc. Natl. Acad. Sci. USA* **85,** 2444-2448.

**Sambrook J. and Russell DW** (2001). *In Molecular cloning-a laboratory manual, Cold Spring Harbor Laboratory Press, 3*^{*rd*} *ed., New York.*

**Schledz M, Seidler A, Beyer P and Neuhaus G** (2001). A novel phytyltransferase from *Synechocystis* sp. PCC 6803 involved in tocopherol biosynthesis. *FEBS Letters* **499,** 15-20.

**Schuep W and Rettenmaier R** (1994). Analysis of vitamin E homologs in plasma and tissue: high-performance liquid chromatography. *Methods Enzymol.* **234,** 294-302.

**Shintani D and DellaPenna D.** (1998). Elevating the vitamin E content of plants through metabolic engineering. *Science* **282,** 2098-2100.

**Soll J. and Schultz G.** (1979). Comparison of geranylgeranyl and phytyl substituted methylquinols in the tocopherol synthesis of spinach chloroplasts. *Biochem. Biophys. Res. Commun.* **91,** 715-720.

**Stocker A, Fretz H, Ruettimann A and Woggon W-D** (1996). The substrate specificity of tocopherol cyclase. *Bioorg. Med. Chem*. **4**, 1129-1134.

**Tanaka R, Oster U, Kruse E, Rüdiger W and Grimm B** (1999). Reduced activity of geranylgeranyl reductase leads to loss of chlorophyll and tocopherol and to partially geranylgeranylated chlorophyll in transgenic tobacco plants expressing antisense RNA for geranylgeranyl reductase. *Plant Physiol.* **120,** 695-704.

**Theriault A, Chao JT, Wang Q, Gapor A and Adeli K** (1999). Tocotrienol: a review of its therapeutic potential. *Clin. Biochem.* **32,** 309-319.

**Qureshi AA, Bradlow BA, Brace L, Manganello J, Peterson DM, Pearce BC Wright JJ, Gapor A and Elson CE** (1995). Response of hypercholesterolemic subjects to administration of tocotrienols. *Lipids* **30,** 1171-1177.

**Wang Q, Theriault A, Gapor A and Adeli K.** (1998). Effects of tocotrienol on the intracellular translocation and degradation of apolipoprotein B: possible involvement of a proteasome independent pathway. *Biochem. Biophys. Res. Com*. **246,** 640-643.

## Claims

1. A polynucleotide that encodes a polypeptide of SEQ NO 1.

2. A polynucleotide comprising at least 30 contiguous bases of SEQ ID NO 1.

3. A polynucleotide having at least 60 % sequence identity to SEQ ID NO 1, wherein the identity is based on the entire coding sequence and is determined by BLAST 2.0 using default parameters.

4. A polynucleotide having at least 60 % sequence identity to SEQ NO 1, wherein the % sequence identity is based on the entire sequence and is determined by GAP using default parameters.

5. A polynucleotide which selectively hybridizes, under stringent conditions and a wash in 2 X SSC at 50 °C, to a hybridization probe derivable from the polynucleotide sequence as set forth in SEQ ID NO 1, or from the genomic sequence represented by SEQ ID NO 1.

6. A polynucleotide complementary to a polynucleotide of claim 5.

7. The polynucleotide of claim 1, wherein the tocotrienol/plastoquinone prenyltransferase polynucleotide is from glycine max, maize, soybean, rice and Arabidopsis thaliana.

8. The polynucleotide of claim 1 encoding a polypeptide, in which 2-metyl-6-geranygeranyl-plastoquinol or 2-methyl-6-solanesyl-plastoquinol is modified.

9. A vector comprising at least one polynucleotide of claim 1.

10. An expression cassette comprising at least one polynucleotide of claim 1 operably linked to a promoter, wherein the polynucleotide is in sense or antisense orientation.

11. A host cell which is introduced with at least one expression cassette of claim 10.

12. The host cell of claim 11 that is a plant cell.

13. A transgenic plant comprising at least one expression cassette of claim 11.

14. The transgenic plant of claim 13, wherein the plant is corn, soybean, sunflower, sorghum, canola, wheat, alfalfa, cotton, rice, barley, millet Arabidopsis thaliana, tomato, Brassica, vegetables, peppers, potatoes, apples, spinach or lettuce.

15. A seed from the transgenic plant of claim 14.

16. The seed of claim 15, wherein the seed is from corn, soybean, sunflower, sorghum, canola, wheat, alfalfa, cooton, rice, barley, millet Arabidopsis thaliana, tomato, Brassica, vegetables, peppers, potatoes, apples, spinach or lettuce.

17. An isolated protein comprising a member selected from the group consisting of:
(a) a polypeptide comprising at least 10 contigous amino acids of SEQ ID NO 2.
(b) a polypeptide which is a plant tocotrienol/tocopherol prenyltransferase
(c) a polypeptide comprising at least 55 % sequence identity to SEQ ID NO 2, wherein the sequence identity is based on the entire sequence and is determined
by BLAST 2.0 using default paramters and has at least one epitope in common with a prenyltransferase.
(d) a polypeptide encoded by a polynucleotide selected from SEQ ID NO 1.
(e) a polypeptide of SEQ ID NO 2.

18. The protein of claim 17, wherein the polypeptide is catalytically active.

19. A ribonucleic acid sequence encoding the protein of claim 18.

20. A method for modulating the level of prenyltransferase protein in a plant, comprising:
(a) stably transforming a plant cell with a prenyltransferase polynucleotide operably linked to a promoter, wherein the polynucleotide is in sense or antisense orientation
(b) growing the plant cell under plant growing conditions to produce a regenerated plant capable of expressing the polynucleotide for a time sufficient to modulate the level of prenyltransferase protein in the plant.

21. The method of claim 20, wherein the prenyltransferase polynucleotide is selected from SEQ ID NO 1.

22. The method of claim 20, wherein the plant is corn, soybean, sunflower, sorghum, canola, wheat, alfalfa, cotton, rice, barley, millet Arabidopsis thaliana, tomato, Brassica, vegetables, peppers, potatoes, apples, spinach or lettuce.

23. The method of claim 20, wherein prenyltransferase is increased.

24. The method of claim 20, wherein prenyltransferase is decreased.

25. A method for modulating the level of tocotrienol in a plant, comprising:
(a) stably transforming a plant cell with a prenyltransferase polynucleotide operably linked to a promoter, wherein the polynucleotide is in sense or anti-sense orientation.
(b) growing the plant cell under plant growing conditions to produce a regenerated plant capable of expressing the polynucleotide for a time sufficient to modulate level of tocopherol in the plant.

26. A method for modulating the level of plastoquinol in a plant, comprising:
(a) stably transforming a plant cell with a prenyltransferase polynucleotide operably linked to a promoter, wherein the polynucleotide is in sense or anti-sense orientation.
(b) growing the plant cell under plant growing conditions to produce a regenerated plant capable of expressing the polynucleotide for a time sufficient to modulate level of plastoquinol in the plant.

27. A method for modulating the level of carotenoids in a plant, comprising:
(a) stably transforming a plant cell with a prenyltransferase polynucleotide operably linked to a promoter, wherein the polynucleotide is in sense or anti-sense orientation.
(b) growing the plant cell under plant growing conditions to produce a regenerated plant capable of expressing the polynucleotide for a time sufficient to modulate level of carotenoid in the plant.

28. The method of claims 25, 26, 27, wherein the prenyltransferase polynucleotide is selected from SEQ ID NO 1.


SEQUENCE LISTING

<110> greenovation Biotech GmbH

<120> Novel Prenyltransferse

<130> Prenyltransferase neu

<140>
<141>

<150> 01129034.3
<151> 2001-12-06

<160> 6

<170> PatentIn Ver. 2.1

<210> 1
<211> 1161
<212> DNA
<213> Arabidopsis thaliana

<220>
<221> CDS
<222> (1)..(1158)

<220>
<221> misc_feature
<222> (75)..(91)
<223> Motive 1

<220>
<221> misc_feature
<222> (464)..(478)
<223> Motive 2

<400> 1
atg gag ctc tcg atc tca caa tca ccg cgt gtt cgg ttc tcg tct ctg 48
Met Glu Leu Ser Ile Ser Gln Ser Pro Arg Val Arg Phe Ser Ser Leu
1               5                   10                  15

gcg cct cgt ttc tta gca gct tct cat cat cat cgt cct tct gtg cat 96
Ala Pro Arg Phe Leu Ala Ala Ser His His His Arg Pro Ser Val His
            20                  25                  30

tta gct ggg aag ttt ata agc ctc cct cga gat gtt cgc ttc acg agc 144
Leu Ala Gly Lys Phe Ile Ser Leu Pro Arg Asp Val Arg Phe Thr Ser

        35                  40                  45

tta tca act tca aga atg cgg tcc aaa ttt gtt tca acc aat tat aga 192
Leu Ser Thr Ser Arg Met Arg Ser Lys Phe Val Ser Thr Asn Tyr Arg
    50                  55                  60

aaa atc tca atc cgg gca tgt tct cag gtt ggt gct gct gag tct gat 240
Lys Ile Ser Ile Arg Ala Cys Ser Gln Val Gly Ala Ala Glu Ser Asp
65                  70                  75                  80

gat cca gtg ctg gat aga att gcc cgg ttc caa aat gct tgc tgg aga 288
Asp Pro Val Leu Asp Arg Ile Ala Arg Phe Gln Asn Ala Cys Trp Arg
                85                  90                  95

ttt ctt aga ccc cat aca atc cgc gga aca gct tta gga tcc act gcc 336
Phe Leu Arg Pro His Thr Ile Arg Gly Thr Ala Leu Gly Ser Thr Ala
            100                 105                 110

ttg gtg aca aga gct ttg ata gag aac act cat ttg atc aaa tgg agt 384
Leu Val Thr Arg Ala Leu Ile Glu Asn Thr His Leu Ile Lys Trp Ser
        115                 120                 125

ctt gta cta aag gca ctt tca ggt ctt ctt gct ctt att tgt ggg aat 432
Leu Val Leu Lys Ala Leu Ser Gly Leu Leu Ala Leu Ile Cys Gly Asn
    130                 135                 140

ggt tat ata gtc ggc atc aat cag atc tac gac att gga atc gac aaa 480
Gly Tyr Ile Val Gly Ile Asn Gln Ile Tyr Asp Ile Gly Ile Asp Lys
145                 150                 155                 160

gtg aac aaa cca tac ttg cca ata gca gca gga gat cta tca gtg cag 528
Val Asn Lys Pro Tyr Leu Pro Ile Ala Ala Gly Asp Leu Ser Val Gln
                165                 170                 175

tct gct tgg ttg tta gtg ata ttt ttt gcg ata gca ggg ctt tta gtt 576
Ser Ala Trp Leu Leu Val Ile Phe Phe Ala Ile Ala Gly Leu Leu Val
            180                 185                 190

gtc gga ttt aac ttt ggt cca ttc att aca agc cta tac tct ctt ggc 624
Val Gly Phe Asn Phe Gly Pro Phe Ile Thr Ser Leu Tyr Ser Leu Gly
        195                 200                 205

ctt ttt ctg gga acc atc tat tct gtt cca ccc ctc aga atg aaa aga 672
Leu Phe Leu Gly Thr Ile Tyr Ser Val Pro Pro Leu Arg Met Lys Arg
    210                 215                 220

ttc cca gtt gca gca ttt ctt att att gcc acg gta cga ggt ttc ctt 720
Phe Pro Val Ala Ala Phe Leu Ile Ile Ala Thr Val Arg Gly Phe Leu

225                 230                 235                 240

ctt aac ttt ggt gtg tac cat gct aca aga gct gct ctt gga ctt cca     768
Leu Asn Phe Gly Val Tyr His Ala Thr Arg Ala Ala Leu Gly Leu Pro
                245                 250                 255

ttt cag tgg agt gca cct gtg gcg ttc atc aca tct ttt gtg aca ctg     816
Phe Gln Trp Ser Ala Pro Val Ala Phe Ile Thr Ser Phe Val Thr Leu
            260                 265                 270

ttt gca ctg gtc att gct att aca aag gac ctt cct gat gtt gaa gga     864
Phe Ala Leu Val Ile Ala Ile Thr Lys Asp Leu Pro Asp Val Glu Gly
        275                 280                 285

gat cga aag ttc caa ata tca acc ctg gca aca aaa ctt gga gtg aga     912
Asp Arg Lys Phe Gln Ile Ser Thr Leu Ala Thr Lys Leu Gly Val Arg
    290                 295                 300

aac att gca ttc ctc ggt tct gga ctt ctg cta gta aat tat gtt tca     960
Asn Ile Ala Phe Leu Gly Ser Gly Leu Leu Leu Val Asn Tyr Val Ser
305                 310                 315                 320

gcc ata tca cta gct ttc tac atg cct cag gtt ttt aga ggt agc ttg     1008
Ala Ile Ser Leu Ala Phe Tyr Met Pro Gln Val Phe Arg Gly Ser Leu
                325                 330                 335

atg att cct gca cat gtg atc ttg gct tca ggc tta att ttc cag aca     1056
Met Ile Pro Ala His Val Ile Leu Ala Ser Gly Leu Ile Phe Gln Thr
            340                 345                 350

tgg gta cta gaa aaa gca aac tac acc aag gaa gct atc tca gga tat     1104
Trp Val Leu Glu Lys Ala Asn Tyr Thr Lys Glu Ala Ile Ser Gly Tyr
        355                 360                 365

tat cgg ttt ata tgg aat ctc ttc tac gca gag tat ctg tta ttc ccc     1152
Tyr Arg Phe Ile Trp Asn Leu Phe Tyr Ala Glu Tyr Leu Leu Phe Pro
    370                 375                 380

ttc ctc tag                                                         1161
Phe Leu
385

<210> 2
<211> 386
<212> PRT
<213> Arabidopsis thaliana

<400> 2
Met Glu Leu Ser Ile Ser Gln Ser Pro Arg Val Arg Phe Ser Ser Leu
1               5                   10                  15

Ala Pro Arg Phe Leu Ala Ala Ser His His His Arg Pro Ser Val His
            20                  25                  30

Leu Ala Gly Lys Phe Ile Ser Leu Pro Arg Asp Val Arg Phe Thr Ser
        35                  40                  45

Leu Ser Thr Ser Arg Met Arg Ser Lys Phe Val Ser Thr Asn Tyr Arg
    50                  55                  60

Lys Ile Ser Ile Arg Ala Cys Ser Gln Val Gly Ala Ala Glu Ser Asp
65                  70                  75                  80

Asp Pro Val Leu Asp Arg Ile Ala Arg Phe Gln Asn Ala Cys Trp Arg
                85                  90                  95

Phe Leu Arg Pro His Thr Ile Arg Gly Thr Ala Leu Gly Ser Thr Ala
            100                 105                 110

Leu Val Thr Arg Ala Leu Ile Glu Asn Thr His Leu Ile Lys Trp Ser
        115                 120                 125

Leu Val Leu Lys Ala Leu Ser Gly Leu Leu Ala Leu Ile Cys Gly Asn
    130                 135                 140

Gly Tyr Ile Val Gly Ile Asn Gln Ile Tyr Asp Ile Gly Ile Asp Lys
145                 150                 155                 160

Val Asn Lys Pro Tyr Leu Pro Ile Ala Ala Gly Asp Leu Ser Val Gln
                165                 170                 175

Ser Ala Trp Leu Leu Val Ile Phe Phe Ala Ile Ala Gly Leu Leu Val
            180                 185                 190

Val Gly Phe Asn Phe Gly Pro Phe Ile Thr Ser Leu Tyr Ser Leu Gly
        195                 200                 205

Leu Phe Leu Gly Thr Ile Tyr Ser Val Pro Pro Leu Arg Met Lys Arg
    210                 215                 220

Phe Pro Val Ala Ala Phe Leu Ile Ile Ala Thr Val Arg Gly Phe Leu
225                 230                 235                 240

Leu Asn Phe Gly Val Tyr His Ala Thr Arg Ala Ala Leu Gly Leu Pro
                245                 250                 255

Phe Gln Trp Ser Ala Pro Val Ala Phe Ile Thr Ser Phe Val Thr Leu
            260                 265                 270

Phe Ala Leu Val Ile Ala Ile Thr Lys Asp Leu Pro Asp Val Glu Gly
        275                 280                 285

Asp Arg Lys Phe Gln Ile Ser Thr Leu Ala Thr Lys Leu Gly Val Arg
    290                 295                 300

Asn Ile Ala Phe Leu Gly Ser Gly Leu Leu Leu Val Asn Tyr Val Ser
305                 310                 315                 320

Ala Ile Ser Leu Ala Phe Tyr Met Pro Gln Val Phe Arg Gly Ser Leu
                325                 330                 335

Met Ile Pro Ala His Val Ile Leu Ala Ser Gly Leu Ile Phe Gln Thr
            340                 345                 350

Trp Val Leu Glu Lys Ala Asn Tyr Thr Lys Glu Ala Ile Ser Gly Tyr
        355                 360                 365

Tyr Arg Phe Ile Trp Asn Leu Phe Tyr Ala Glu Tyr Leu Leu Phe Pro
    370                 375                 380

Phe Leu
385

<210> 3
<211> 36
<212> DNA
<213> Artificial Sequence

<220>
<223> Description of Artificial Sequence: Primer PQ5

<400> 3
aagcttctgg agctgtcgat ctcacaatca ccgcgt                              36

<210> 4
<211> 36
<212> DNA
<213> Artificial Sequence

<220>

<223> Description of Artificial Sequence: Primer PQ3

<400> 4
catatcgtcg acatgaaatt gaaagctaga ggaagg                                36

<210> 5
<211> 36
<212> DNA
<213> Artificial Sequence

<220>
<223> Description of Artificial Sequence: Primer PQBY5

<400> 5
gcccttcccg ggatggagct gtcgatctca caatca                                36

<210> 6
<211> 36
<212> DNA
<213> Artificial Sequence

<220>
<223> Description of Artificial Sequence: Primer PQBY3

<400> 6
tgaaatgaat tcctagagga aggggaataa cagata                                36